(19) **Europäisches Patentamt**

**European Patent Office**

**Office européen des brevets**

(11) **EP 1 361 284 A1**

(12) **EUROPEAN PATENT APPLICATION**

(43) Date of publication:
**12.11.2003 Bulletin 2003/46**

(51) Int Cl.⁷: **C12Q 1/37**, C12N 9/50

(21) Application number: **02010576.3**

(22) Date of filing: **10.05.2002**

(84) Designated Contracting States:
**AT BE CH CY DE DK ES FI FR GB GR IE IT LI LU MC NL PT SE TR**
Designated Extension States:
**AL LT LV MK RO SI**

(71) Applicant: **Direvo Biotech AG**
**50829 Köln (DE)**

(72) Inventors:
- **Koltermann, André**
  **50829 Köln (DE)**
- **Kettling, Ulrich**
  **50829 Köln (DE)**
- **Haupts, Ulrich**
  **50829 Köln (DE)**

- **Tebbe, Jan**
  **50829 Köln (DE)**
- **Scholz, Peter**
  **50829 Köln (DE)**
- **Pilling, Jens**
  **50829 Köln (DE)**
- **Werner, Susanne**
  **50829 Köln (DE)**
- **Rarbach, Markus**
  **50829 Köln (DE)**

(74) Representative:
**Helbing, Jörg, Dr. Dipl.-Chem. et al**
**Patentanwälte**
**von Kreisler-Selting-Werner,**
**Postfach 10 22 41**
**50462 Köln (DE)**

(54) **Process for generating sequence-specific proteases by directed evolution and use thereof**

(57)     A process for generating sequence-specific proteases by screening-based directed evolution is disclosed. The use of the process provides proteases recognizing and cleaving user-definable amino-acid sequences with high sequence-specificity. Proteases obtainable by the process can be used in a variety of medical, diagnostic and industrial applications.

Fig. 1

## Description

A process for generating sequence-specific proteases by screening-based directed evolution is disclosed. The use of the process provides proteases recognizing and cleaving user-definable amino-acid sequences with high sequence-specificity. Proteases obtainable by the process can be used in a variety of medical, diagnostic and industrial applications.

## Background of the Invention

Proteolytic enzymes or proteases are a class of enzymes which has an outstanding position among the different enzymes, since the reaction which proteases catalyse is the cleavage of peptide bonds in other proteins. Proteases are not only very common enzymes in nature, but also belong to the most important enzymes for medical and industrial use. Of the total worldwide sales of enzymes, which is estimated to be more than USD 1 billion per year, proteases account for approximately 60%. Based on the functional group present at the active site, proteases are classified into four groups, i.e., serine proteases (EC 3.4.21), cysteine proteases (EC 3.4.22), aspartic proteases (EC 3.4.23), and metalloproteases (EC 3.4.24). Classification into one of the four groups is typically done by experimental determination of sensitivity towards different types of protease inhibitors. Furthermore, proteases of the four groups differ in their biochemical properties. For example, serine proteases are sensitive to inhibitors 3,4-DCI, DFP, PMSF and TLCK, and have an pH optimum between pH 7 and 11. Aspartic proteases are inhibited by pepstatin, DAN and EPNP, and predominantly have a pH optimum between pH 3 and 4. Cysteine proteases are sensitive to sulfhydryl inhibitors such as PCMB, and besides a few exceptions have neutral pH optima. Metalloproteases are characterized by the requirement of a divalent metal ion for their activity. Therefore, metalloproteases are inhibited by chelating agents such as EDTA, and have neutral or alkaline pH optima. Among these four groups, further classification is usually done on the basis of structural similarities.

Besides such a combined biochemical and structural classification, proteases can be grouped according to their substrate spectrum. The two most general groups to be distinguished are exoproteases and endoproteases. Exoproteases only cleave peptide bonds at the very end of a peptide, whereas endoproteases catalyze the cleavage of bonds anywhere in a peptide strand. The specificity of proteases, i.e. their ability to recognize and hydrolyze specifically certain peptide substrates while others remain uncleaved, can be expressed qualitatively and quantitatively. Qualitative specificity refers to the kind of amino acid residues that are accepted by a protease at certain positions of the peptide substrate. For example, trypsin and the tissue-type plasminogen activator are related with respect to their qualitative specificity, since both of them require at the position P1 an arginine or a similar residue (nomenclature of peptide substrate positions according to the nomenclature of Schlechter & Berger (Biochem. Biophys. Res. Commun. 27 (1967) 157-162)). On the other hand, quantitative specificity refers to the relative number of peptide substrates that are accepted as substrates. The quantitative specificity can be expressed by the term

$$s = - \log(Q),$$

where Q is the ratio of all accepted peptide substrates versus all possible peptide substrates. Quantitative specificities of several proteases are shown exemplarily in Table I. The calculation of quantitative specificities bases on the twenty naturally occurring amino acids only, and on the assumption that all combinations of these twenty amino acids are feasible. Consequently, proteases that accept only a small portion of all possible peptides have a high specificity, whereas the specificity of proteases that - to an extreme - cleave any peptide substrate would theoretically be zero.

Table I:

| Quantitative specificities of different proteases | | | | | | | | | | | |
|---|---|---|---|---|---|---|---|---|---|---|---|
| protease | Substrate requirements | | | | | | | | | Quantitative specificity | |
| | P6 | P5 | P4 | P3 | P2 | P1 | P1' | P2' | P3' | Q | s = -log Q |
| | | | | | | | | | | | |
| | x | x | x | x | x | x | x | x | x | 1.00E+00 | 0 |
| Chymotrypsin | x | x | x | x | x | F/Y/W | x | x | x | 1.50E-01 | 0.82 |
| Papain | x | x | x | x | F/V/L | x | x | x | x | 1.50E-01 | 0.82 |

Table I:   (continued)

| Quantitative specificities of different proteases | | | | | | | | | | | |
|---|---|---|---|---|---|---|---|---|---|---|---|
| protease | Substrate requirements | | | | | | | | | Quantitative specificity | |
| | P6 | P5 | P4 | P3 | P2 | P1 | P1' | P2' | P3' | Q | s = -log Q |
| Trypsin | x | x | x | x | x | K/R | x | x | x | 1.00E-01 | 1.00 |
| Pepsin | x | x | x | x | x | F/Y/L | W/F/Y | x | x | 2.25E-02 | 1.65 |
| TEV | E | x | x | Y | x | Q | S/G | x | x | 1.25E-05 | 4.90 |
| Plasmin | x | x | K/V/I/F | x | F/Y/W | R/K | N | A | x | 7.50E-06 | 5.12 |
| thrombin | x | x | L/I/V/F | x | P | R | N | A | x | 1.25E-06 | 5.90 |
| t-PA | x | x | C | P | G | R | V | V | G | 7.81E-10 | 9.11 |
| (Amino acid residues are abbreviated as shown in Table II. X refers to any amino acid residue.) | | | | | | | | | | | |

The quantitative specificity of proteases varies over a wide range. There are very unspecific proteases known, such as papain which cleaves all polypeptides that contain a phenylalanine, a valine or a leucine residue (s = 0.82), or trypsin which cleaves all polypeptides that contain an arginine or a lysine residue (s = 1.0). On the other hand, there are highly specific proteases known, such as the tissue-type plasminogen activator (t-PA) which cleaves plasminogen only at a single specific sequence (s = 9.11). Proteases with high substrate specificity play an important role in the regulation of protein functions in living organisms. The specific cleavage of polypeptide substrates, for example, activates precursor proteins or deactivates active proteins or enzymes, thereby regulating their functions. Several proteases with high substrate specificities are used in medical applications. Pharmaceutical examples for activation or deactivation by cleavage of specific polypeptide substrates are the application of t-PA in acute cardiac infarction which activates plasminogen to resolve fibrin clots, or the application of Ancrod in stroke which deactivates fibrinogen, thereby decreasing blood viscosity and enhancing its transport capacity. While t-PA is a human protease with an activity necessary in human blood regulation, Ancrod is a non-human protease. It was isolated from the viper Agkistrodon rhodostoma, and comprises the main ingredient of the snake's poison. Therefore, there exist a few non-human proteases with therapeutic applicability. Their identification, however, is usually highly incidental.

The treatment of diseases by administering drugs is typically based on a molecular mechanism initiated by the drug that activates or inactivates a specific protein function in the patient's body, be it an endogenous protein or a protein of an infecting microbe or virus. While the action of chemical drugs on these targets is still difficult to understand or to predict, protein drugs are able to specifically recognize these target proteins among millions of other proteins. Prominent examples of proteins that have the intrinsic possibility to recognize other proteins are antibodies, receptors, and proteases. Although there are a huge number of potential target proteins, only very few proteases are available today to address these target proteins. Due to their proteolytic activity, proteases are particularly suited for the inactivation or activation of protein targets. When considering human proteins only, the number of potential target proteins is yet enormous. It is estimated that the human genome comprises between 30,000 and 100,000 genes, each of which encodes a different protein. Many of these proteins are involved in human diseases and are therefore potential pharmaceutical targets. Proteases recognizing and cleaving these target proteins with a high specificity are consequently of high value as potential drugs. The medical application of such proteases, however, is restricted by their occurrence. For example, there are theoretically 25 billion different possibilities for a specificity of s = 10.4 (corresponding to the specific recognition of a unique sequence of eight amino acid residues). It is highly unlikely to find such a protease with one particular qualitative specificity by screening natural isolates.

Laboratory techniques to generate proteolytic enzymes with altered sequence specificities are in principle known. They can be classified by their expression and selection systems. Genetic selection means to produce inside an organism a protease which is able to cleave a precursor protein which in turn results in an alteration of the growth behavior of the producing organism. From a population of organisms with different proteases those can be selected which have an altered growth behavior. This principle was reported by Davis et al. (US 5258289, WO 96/21009). The production of a phage system is dependent on the cleavage of a phage protein which only can be activated in the presence of a proteolytic enzyme or antibody which is able to cleave the phage protein. Selected proteolytic enzymes or antibodies would have the ability to cleave an amino acid sequence for activation of phage production. Furthermore, there is no control of the specificity of the proteases that are selected. The system does not select for proteases with low activities for other peptides than the used peptide substrate. Additionally, this system does not allow a precise characterization of the kinetic constants of the selected proteases ($k_{cat}$, $K_M$). Several other systems with intracellular protease expression are reported but they all suffer from the disadvantages mentioned above. Some of them use a genetic reporter system which allows a selection by screening instead of a genetic selection, but also cannot overcome the intrinsic insufficiency of the intracellular characterization of proteases.

A system to generate proteolytic enzymes with altered sequence specificities with membrane-bound proteases is reported. Iverson et al. (WO 98/49286) describe an expression system for a membrane-bound protease which is displayed on the surface of cells. An essential element of the experimental design is that the catalytic reaction has to be performed at the cell surface, i.e., the substrates and products must remain associated with the bacterium expressing the enzyme at the surface. This restriction limits the generation of proteolytic enzymes with altered sequence specificities and does not allow a precise characterization of the kinetic constants of the selected proteases ($k_{cat}$, $K_M$). Furthermore, the method does not allow the control of the position at which the peptide is cleaved. Additionally, positively identified proteases will have the ability to cleave a certain amino acid (aa) sequence but they also may cleave many other aa sequences. Therefore, there is no control of the specificity of the proteases that are selected.

A system to generate proteolytic enzymes with altered sequence specificities with self-secreting proteases is also reported. Duff et al. (WO 98/11237) describe an expression system for a self-secreting protease. An essential element of the experimental design is that the catalytic reaction acts on the protease itself by an autoproteolytic processing of the membrane-bound precursor molecule to release the matured protease from the cellular membrane into the extracellular environment. Therefore, a fusion protein must be constructed where the target peptide sequence replaces the natural cleavage site for autoproteolysis. Limitations of such a system are that positively identified proteases will have the ability to cleave a certain aa sequence but they also may cleave many other peptide sequences. Therefore, high substrate specificity can not be achieved with such an approach. Additionally, such a system is not able to control that selected proteases cleave at a specific position in a defined aa sequence and it does not allow a precise characterization of the kinetic constants of the selected proteases ($k_{cat}$, $K_M$).

Most of these approaches apply methods of directed evolution for the generation of proteolytic enzymes with altered sequence specificities. Several different mutation and recombination methods to generate genetic libraries are reported and described elsewhere. All the different methods suffer from their lack of precise selection of positive protease variants from large libraries. First, these methods are not able to distinguish between single and multi turn-overs of peptide substrates which is necessary in order to prevent the selection of low $k_{cat}$ variants. Secondly, it is not possible to trigger enzyme and substrate concentration to select protease variants for lower $K_M$. Third, none of these systems allows the selection of a protease with an increased activity on the desired peptide substrate whereby the activity on the original peptide substrate decreases.

Methods which fulfill the above mentioned three selection criteria ($k_{cat}$, $K_M$ and substrate specificity) for generating proteolytic enzymes with high sequence-specificity applying screening-based directed evolution have hitherto not be made available.

**Summary of the Invention**

Thus, the technical problem underlying the present invention is to provide a method for generating new proteases with user-defined substrate specificities by applying directed evolution. In particular, the invention is directed to a method for the evolution of novel proteases towards selective recognition and cleavage of specific amino-acid sequences only. This technical problem has been solved by the embodiments of the invention specified below and in the appended claims. The present invention is thus directed to

(1) a method for generating sequence-specific proteases with target substrate specificities which comprises the following steps

(a) providing a population of proteases comprised of variants of one first protease or of variants or chimeras of two or more first proteases, said first proteases having a substrate specificity for a particular amino acid sequence of a first peptide substrate;
(b) contacting said population of proteases with one or more second substrates, comprising at least one specific amino acid sequence resembling the amino acid sequence of the target peptide substrate but being not present within the first peptide substrate; and
(c) selecting one or more protease variants from the population of proteases provided in step (a) having specificity for said specific amino acid sequence of the second substrates provided in step (b) under conditions that allow identification of proteases that recognize and hydrolyse preferably said specific one amino acid sequence within the second substrates;

(2) in a preferred embodiment of (1) above only one second substrate is used in the one or more cycles (a) to (c), i.e., the second substrate is identical with the target substrate;
(3) in a further preferred embodiment of (1) above different second substrates are used, and the second substrates have an intermediate character with regard to the first substrate and the target substrate, and the last second substrate that is used is identical with the target substrate;

(4) in a particular preferred embodiment of (1) to (3) above the target protease has a specificity similar to tissue-type plasminogen activator and cleaves the target substrate CPGR$^{\downarrow}$VVGG; and

(5) the protease obtainable by the method of (1) to(4) above, preferably by the method of (4) above.

The identification and selection of proteases that have evolved towards the target specificity is done by screening for catalytic activities on different peptide substrates, either by screening for increased affinity, or by using two or more substrates in comparison, or by using unspecific peptides as competitors, or by using intermediate peptide substrates.

The following detailed description will disclose the preferred features, advantages and the utility of the present invention.

**Brief Description of the Figures**

The following figures are provided in order to explain further the present invention in supplement to the detailed description:

Figure 1    depicts schematically the two alternatives A and B of the method of the invention.

Figure 2    distinguishes the two alternatives A and B of the method of the invention by showing schematically the qualitative and quantitative changes in specificity during evolution towards the target specificity.

Figure 3    illustrates schematically how proteases with changed catalytic activities are evolved using the two alternatives A and B of the method of the invention.

Figure 4    depicts schematically in two different forms the intermediate approach as one particular aspect of the invention that uses intermediate substrates.

Figure 5    illustrates schematically how, according to the invention, proteases with changed catalytic activities are evolved using the intermediate approach.

Figure 6    shows exemplarily an expression vector for S. cerevisiae that can be used for the method of the invention.

Figure 7    shows exemplarily the hydrolysis of a peptide substrate by the tobacco etch virus protease.

Figure 8    shows exemplarily a distribution of catalytic activities obtained by screening using confocal fluorescence spectroscopy.

Figure 9    shows exemplarily the decrease in $K_M$ during evolution towards higher affinity.

Figure 10    shows exemplarily the change in specificity during evolution of proteases towards the specificity of t-PA.

Figure 11    depicts schematically a preferred variant of the intermediate approach.

**Detailed Description of the Invention and of the Figures**

In the framework of this invention the following terms and definitions are used.

The term "protease" means any protein molecule acting in the hydrolysis of peptide bonds. It includes naturally-occurring proteolytic enzymes, as well as variants thereof obtained by site-directed or random mutagenesis or any other protein engineering method, any fragment of an proteolytic enzyme, or any molecular complex or fusion protein comprising one of the aforementioned proteins. A "chimera of proteases" means a fusion protein out of two or more fragments derived from different parent proteases.

The term "substrate" or "peptide substrate" means any peptide, oligopeptide, or protein molecule of any amino acid composition, sequence or length, that contains a peptide bond that can be hydrolyzed catalytically by a protease. The peptide bond that is hydrolyzed is referred to as the "cleavage site". Numbering of positions in the substrate is done according to the system introduced by Schlechter & Berger (Biochem. Biophys. Res. Commun. 27 (1967) 157-162). Amino acid residues adjacent N-terminal to the cleavage site are numbered P1, P2, P3, etc., whereas residues adjacent C-terminal to the cleavage site are numbered P1', P2', P3', etc.

The term "specificity" means the ability of a protease to recognize and hydrolyze selectively certain peptide substrates while others remain uncleaved. Specificity can be expressed qualitatively and quantitatively. "Qualitative specificity" refers to the kind of amino acid residues that are accepted by a protease at certain positions of the peptide substrate. "Quantitative specificity" refers to the number of peptide substrates that are accepted as substrates. Quantitative specificity can be expressed by the term s, which is the negative logarithm of the number of all accepted peptide substrates divided by the number of all possible peptide substrates. Proteases that accept only a small portion of all possible peptide substrates have a "high specificity" (s » 1). Proteases that accept almost any peptide substrate have a "low specificity". Proteases with very low specificity (s ≤ 1) are also referred to as "unspecific proteases".

The term "first protease" describes any protease used in step (a) of this invention as the starting point in order to generate populations of protease variants that are related to this first protease. The term "first substrate" or "first peptide substrate" describes a substrate that is recognized and hydrolyzed by the first protease. The term "first specificity" describes the qualitative and quantitative specificity of the first protease.

The term "evolved protease" describes any protease that is generated by use of the method of the invention. The term "target substrate" or "target peptide substrate" describes a substrate that is recognized and hydrolyzed by the evolved protease. The term "target specificity" describes the qualitative and quantitative specificity of the evolved protease that is to be generated by use of the method of the invention. Thus, the target specificity defines the specificity of the evolved protease for the target peptide substrate while other substrates are not or very weakly recognized and hydrolyzed.

The term "intermediate" or "intermediate substrate" describes any substrate that has an intermediate character between two other substrate. The intermediate character can base on the amino acid composition, the amino acid sequence, the properties of the amino acid residues contained in the substrates, or a combination of these characteristics.

Catalytic properties of proteases are expressed using the kinetic parameters "$K_M$" or "Michaelis Menten constant", "$k_{cat}$" or "catalytic rate constant", and "$k_{cat}/K_M$" or "catalytic efficiency", according to the definitions of Michaelis and Menten (Fersht, A., Enzyme Structure and Mechanism, W. H. Freeman and Company, New York, 1995). The term "catalytic activity" describes the rate of conversion of the substrate under defined conditions.

Amino acids are abbreviated according to the following Table II either in one- or in three-letter code.

Table II:

| Amino acid abbreviations | | |
|---|---|---|
| Abbreviations | | Amino acid |
| A | Ala | Alanin |
| C | Cys | Cysteine |
| D | Asp | Aspartic cid |
| E | Glu | Glutamic acid |
| F | Phe | Phenylalanine |
| G | Gly | Glycine |
| H | His | Histidine |
| I | Ile | Isoleucine |
| K | Lys | Lysine |
| L | Leu | Leucine |
| M | Met | Methionine |
| N | Asn | Asparagine |
| P | Pro | Proline |
| Q | Gln | Glutamine |
| R | Arg | Arginine |
| S | Ser | Serine |
| T | Thr | Threonine |
| V | Val | Valine |
| W | Trp | Tryptophane |
| Y | Tyr | Tyrosine |

As set forth above, the present invention is directed to a method for generating sequence-specific proteases with a target substrate specificity by applying principles of molecular evolution. According to the invention, this is achieved by providing a population of proteases being related to each other, as well as a peptide substrate that resembles the target substrate, and selecting one or more protease variants from the population of proteases with respect to their specificity for the provided substrate. The selection is done under conditions that allow identification of proteases that recognize and hydrolyze the target sequence preferably.

In particular, embodiment (1) of the invention relates to a method for generating sequence-specific proteases with target substrate specificities, wherein the following steps are carried out:

(a) providing a population of proteases, wherein each variant is related to one or more first proteases, these first proteases having a first substrate specificity;
(b) providing one or more peptide substrates comprising at least one amino-acid sequence that resembles the target peptide substrate;
(c) selecting one or more protease variants from the population of proteases provided in step (a) with respect to their specificity for the substrate provided in step (b) under conditions that allow identification of proteases that recognize and cleave the target sequence preferably;

and wherein steps (a) to (c) are carried out cyclically until one or more protease variants with the target substrate specificity are identified.

When repeating steps (a) to (c), the one or more proteases selected in step (c) of one cycle are used as the one or more first proteases in step (a) of the next cycle.

In one alternative of the invention, the one or more first proteases serving as starting points in step (a) of the method have a high sequence specificity which is maintained high during the directed evolution towards the target specificity.

In another alternative of the method, the one or more first proteases serving as starting points in step (a) of the method have a low sequence specificity, which is increased during the directed evolution towards the target specificity.

The steps (a) to (c) of the above method are carried out for at least one cycle. Preferably, however, these steps are carried out for several cycles, with each one or more protease variants selected in one cycle being the origin of the population of protease variants in the next cycle. Preferably, more than one and less than hundred, more preferably more than two and less than fifty, particularly preferably more than three and less than twenty, especially preferably more than four and less than ten, and most preferably five cycles of steps (a) to (c) are carried out until one or more protease variants with the target substrate specificity are identified.

The invention applies evolutionary means as described in very detail in patent application WO9218645 with that document being incorporated in its entirety for all purposes.

For an overview on the application of evolutionary principles to molecular biotechnology, which is usually referred to as "directed evolution" or "evolutionary biotechnology", see the review by Koltermann & Kettling (Biophys. Chem. 66 (1997) 159-177).

Part of the invention is the provision of populations of protease variants wherein each variant is related to one or more first proteases. In principle, there can be a large number of these first proteases, all together being the origin for the first cycle of the method. It is preferred, however, that these first proteases comprise fifty or less different proteases, more preferably ten or less different proteases, especially preferably two or less different proteases. Most preferably, only one first protease is employed.

According to the invention, any protease can be used as first protease. Preferably, an endoprotease is used as first protease. It is preferred that the protease belongs to the group of proteases consisting of Serine proteases (EC 3.4.21), Cysteine proteases (EC 3.4.22), Aspartic proteases (EC 3.4.23), and Metalloproteases (EC 3.4.24). First proteases are characterized by their ability to recognize and hydrolyze peptide substrates with a certain qualitative and quantitative specificity. First proteases can have a specificity in the same range as the specificity of the protease that is to be generated. Examples for proteases with relatively high specificities are TEV protease, HIV-1 protease, BAR1 protease, Factor Xa, Thrombin, tissue-type plasminogen activator, Kex2 protease, TVMV-protease, RSV protease, MuLV protease, MPMV protease, MMTV protease, BLV protease, EIAV protease, SIVmac protease. Alternatively, the first proteases have a lower specificity than the specificity of the protease that is to be generated. As an extreme example of the latter, proteases with very low sequence specificity are employed, for example proteases such as Papain, Trypsin, Chymotrypsin, Subtilisin, SET (trypsin-like serine protease from Streptomyces erythraeus), Elastase, Cathepsin G or Chymase.

The provision of populations of proteases is essentially done as described in patent application WO9218645. According to the invention, genes encoding protease variants are ligated into a suitable expression vector by standard molecular cloning techniques (Sambrook, J.F; Fritsch, E.F.; Maniatis,T.; Cold Spring Harbor Laboratory Press, Second Edition, 1989, New York). The vector is introduced in a suitable expression host cell, which expresses the corresponding protease variant. Particularly suitable expression hosts are bacterial expression hosts such as Escherichia coli or Bacillus subtilis, or yeast expression hosts such as Saccharomyces cerevisae or Pichia pastoris, or mammalian expression hosts such as Chinese Hamster Ovary (CHO) or Baby Hamster Kidney (BHK) cell lines, or viral expression systems such as the Baculovirus system. Alternatively, systems for *in vitro* protein expression can be used.

In a preferred embodiment of the invention, the genes are ligated into the expression vector behind a suitable signal sequence that leads to secretion of the protease variants into the extracellular space, thereby allowing direct detection of protease activity in the cell supernatant. Particularly suitable signal sequences for Escherichia coli are HlyA, for Bacillus subtilis AprE, NprB, Mpr, AmyA, AmyE, Blac, SacB, and for S. cerevisiae Bar1, Suc2, Matα, Inu1A, Ggplp.

In another preferred embodiment of the invention, the protease variants are expressed intracellularly and the peptide substrates are expressed also intracellularly. Preferably, this is done essentially as described in patent application WO 0212543, using a fusion peptide substrate comprising two autofluorescent proteins linked by the substrate amino-acid sequence.

In another preferred embodiment of the invention, the protease variants are expressed intracellularly, or secreted into the periplasmatic space using signal sequences such as DsbA, PhoA, PelB, OmpA, OmpT or gIII for Escherichia coli, followed by permeabilisation or lysis step to release the protease variants into the supernatant. The destruction of the membrane barrier can be forced by the use of mechanical means such as ultrasonic, French press, or the use of membrane-digesting enzymes such as lysozyme.

As a further alternative, the genes encoding the protease variants are expressed cell-free by the use of a suitable cell-free expression system. In a particularly preferred embodiment, the S30 extract from Escherichia coli cells is used for this purpose as described by Lesly et al. (Methods in Molecular Biology 37 (1995) 265-278).

The relatedness to the one or more first proteases can be achieved by several procedures. For example, the genes encoding the one or more first proteases are modified by methods for random nucleic acid mutagenesis. In a preferred embodiment of the invention, random mutagenesis is achieved by the use of a polymerase as described in patent WO 9218645. According to this embodiment, the one or more genes encoding the one or more first proteases are amplified by the use of a polymerase with a high error rate, or under conditions that increase the rate of misincorporations, thereby leading to a population of genes wherein each gene encodes a protease that is related to the one or more first proteases. For example the method according to Cadwell, R.C and Joyce, G.F. can be employed (PCR Methods Appl. 2 (1992) 28-33). Other methods for random mutagenesis that can be employed make use of mutator strains, UV-radiation or chemical mutagens. Most preferably, errors are introduced into the gene at or near but below the error threshold as described in patent specification WO 9218645.

In another preferred embodiment of the invention, certain parts of the gene encoding the protease variants are randomized completely with respect to the amino-acid sequence, and are re-introduced into the gene as an oligonucleotide cassette. This technique is usually referred to as cassette mutagenesis (Oliphant, A.R. et al. Gene 44 (1986) 177-183; Horwitz, M.S., et al. Genome 31 (1989) 112-117). In a particularly preferred embodiment of the invention, the part of the gene that encodes amino acid residues that are essential for recognition of the substrate is randomized via cassette mutagenesis. These residues can be identified from structural studies. In particular, residues comprising parts of the substrate binding pocket are targeted by cassette mutagenesis. Alternatively, substituting each amino acid residue with an alanine, and analyzing whether there is an effect on the or the catalytic activity can identify such residues. As a further alternative, these residues can be identified by first introducing random mutations into the gene, screening for an effect on specificity, affinity, or catalytic activity, and determining afterwards the position of mutations in variants that represent altered specificity, affinity or altered catalytic activity. As an extreme of this approach, the completely randomized sequence can have the length of one nucleotide only. This approach is typically referred to as site saturation mutagenesis.

In a further preferred embodiment of the invention, methods for homologous in-vitro recombination are used for the provision of protease populations. Examples of methods that can be applied are the Recombination Chain Reaction according to patent application WO 0134835, the DNA-Shuffling method according to the patent application WO 9522625, the Staggered Extension method according to patent WO 9842728, or the Random Priming recombination according to patent application WO9842728. Furthermore, also methods for non-homologous recombination such as the Itchy method can be applied (Ostermeier, M. et al. Nature Biotechnology 17 (1999) 1205-1209).

In further embodiments of the invention, the above-mentioned methods are combined with each other. In a particularly preferred embodiment, the Recombination Chain Reaction is combined with random mutagenesis such as error-prone PCR according to Cadwell, R.C and Joyce, G.F. (PCR Methods Appl. 2 (1992) 28-33) in order to de-couple mutations selected in the round before and to introduce simultaneously a defined number of new random mutations into the population.

The coupling of protease genotype and phenotype is achieved by use of sample carriers that enable compartmentation of samples, and the distribution of genotypes into sample carriers is done at a multiplicity per compartment that allows sufficient differentiation of phenotypes.

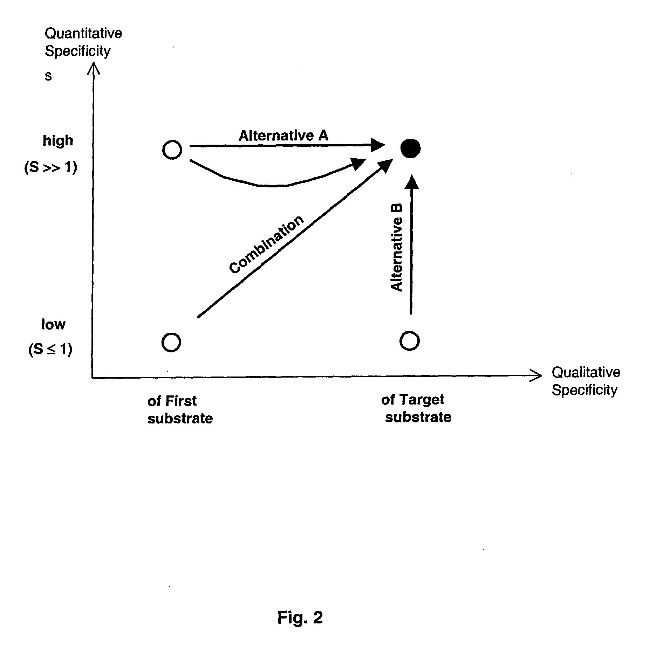

The one or more first proteases that serve as the starting point of the method either have a specificity which is in the range of the target specificity that is to be generated by the method, or have a lower specificity than the target specificity. Accordingly, the method of the invention is either performed under conditions that maintain the specificity quantitatively and alters it qualitatively (Alternative A), or the method of the invention is performed under conditions that maintains the specificity qualitatively and increases it quantitatively (Alternative B). Moreover, both approaches can be combined. These three principle alternatives are shown schematically in Figure 2.

In a preferred embodiment of the invention corresponding to alternative A, the one or more first proteases have a first specificity that is quantitatively in the range of the target specificity, but qualitatively distinct from the target specificity. Proteases having the target substrate specificity are achieved using the method of the invention by selecting protease variants under conditions that allow identification of proteases that recognize and cleave the target sequence preferably.

In another preferred embodiment of the invention corresponding to alternative B, the specificity of the one or more first proteases is quantitatively lower when compared to the target specificity. This means that they accept and hydrolyze a larger number of peptide substrates. This low first specificity is subsequently increased by the method of the invention until it is in the range of the target specificity. As a preferred variant of this embodiment, the first specificity is qualitatively related to the target specificity. Thus, the large number of peptide substrates that is accepted and hydrolyzed includes the target substrate already. Accordingly, amino acid residues that are essential in the first substrate remain essential residues in the target substrate. Then, proteases having the target substrate specificity are achieved using the method of the invention by selecting protease variants under conditions that allow identification of proteases that recognize and cleave the target sequence preferably.

Another part of the invention is the provision of peptide substrates that resemble the target substrate, and the use of these substrates for screening of protease variants with respect to their catalytic activity.

In a preferred embodiment of the invention, suitable peptide substrates are synthesized via the solid phase peptide synthesis approach of Merrifield et al (Nature. 207 (1965) 522-523).These peptide substrates are then incubated for a certain time in a sample buffer containing the protease variant to be tested. The hydrolysis of the peptide is then analyzed by a suitable method. For example, the amount of fragmented peptides can be analyzed by chromatography. In particular, peptide fragments are analyzed advantageously on a reversed phase HPLC system. Alternatively, the peptide substrate is modified in any way to enable the analysis of peptide hydrolysis. In particular, the peptide substrate may carry functional groups that enable the detection of the hydrolysis of the substrate. Such functional groups include, but are not limited to, the following:

one or more fluorophores or chromophores, whose spectroscopic properties change upon hydrolysis of the peptide, whereby screening is performed through determination of the change in spectroscopic properties; or

two fluorophores which are distinguishable by their fluorescence properties and which are attached to opposite ends of the second substrate, whereby the screening is performed through confocal fluorescence spectroscopy at fluorophore concentrations below 1 μM; or

two fluorophores which form a fluorescence resonance energy transfer (FRET) pair and which are attached to opposite ends of the second substrate, whereby screening is performed through determination of the decrease in the energy transfer between the two fluorophores; or

a first and second autofluorescent protein flanking the second substrate, whereby the screening is performed through confocal fluorescence spectroscopy at substrate concentrations below 1 μM; or

a fluorophore and a quencher molecule which are attached to opposite ends of the second substrate, whereby screening is performed through determination of the decrease in quenching of the fluorophore; or

a fluorophor or a chromophor and a binding moiety which are attached to opposite ends of the second substrate, whereby screening is performed through determination of binding of the binding moiety to a specific binding partner; or

a radioactive label and a binding moiety which are attached to opposite ends of the second substrate, whereby screening is performed through use of a scintillation proximity assay; or

any combination thereof.

With respect to the above mentioned functional groups, a chemical group can be attached to the peptide that alters its properties when the peptide is hydrolyzed.

For example, a para-nitrophenyl group can be used for this purpose. As another example, one or more fluorophores and/or a quencher molecule are attached to the peptide, and the amount of fragmented peptide is analysed by measuring a difference in the fluorescence of the fluorophors. For example, two fluorophores that are suited to form a FRET (fluorescence resonance energy transfer) pair are attached to the peptide at opposite ends, and the hydrolysis of the peptide is measured by a decrease in the energy transfer between the two fluorophors. For example, Rhodamine Green (Molecular Probes Inc., Oregon, USA) and Tetramethylrhodamine (Molecular Probes Inc., Oregon, USA) can be used as fluorophors that are suited to form such a FRET pair.

In a particularly preferred embodiment of the invention, two fluorophores that do not form a substantial FRET pair are attached to opposite ends of synthetic peptide substrates. As an example, Rhodamine Green (Molecular Probes Inc., Oregon, USA) and Cy-5 (Amersham Biosciences Europe GmbH, Freiburg) can be used for this purpose, and covalent attachment of the dye can be achieved via a succinimidyl ester linkage to a primary amino group of the peptide. Hydrolysis of these peptides is preferably analysed by means of confocal fluorescence spectroscopy according to patent applications WO 9416313 and WO9613744, which are hereby incorporated by reference in their entirety for all purposes. Due to the high sensitivity of confocal fluorescence spectroscopy, substrates are used in concentrations below one micromolar, more preferably below hundred nanomolar, and most preferably below ten nanomolar. Therefore, screening according to this embodiment is done substantially below the $K_M$ of typical proteases.

In another particularly preferred embodiment of the invention, fusion proteins comprising a first autofluorescent protein, a peptide, and a second autofluorescent protein are used as peptide substrates. According to patent application WO0212543, which is hereby incorporated by reference in its entirety for all purposes, autofluorescent include the Green Fluorescent Protein GFP and its mutants, as well as dsRED and its mutants. Fusion proteins can be produced by expression of a suitable fusion gene in E. coli, lysis of cells and purification of the fusion protein by standard methods such as ion exchange chromatography or affinity chromatography.

It is an essential part of the invention that proteases with the target substrate specificity are generated by selecting protease variants under conditions that allow identification of proteases that recognize and cleave the target sequence preferably. This selection can be achieved according to the different aspects of the invention as outlined below.

In a first aspect of the invention, proteases that recognize and cleave the target sequence preferably are identified by screening for proteases with a high affinity for the target substrate sequence. High affinity corresponds to a low $K_M$ which is selected by screening at target substrate concentrations substantially below the $K_M$ of the first protease. This aspect is referred to as the "affinity approach".

In a preferred embodiment of this aspect of the invention, the peptide substrate provided in step (b) is linked to one or more fluorophores that enable the detection of the hydrolysis of the peptide substrate at concentrations below 10 μM, preferably below 1 μM, more preferably below 100 nM, and most preferably below 10 nM.

In a second aspect of the invention, proteases that recognize and cleave the target sequence only are identified by providing two or more peptide substrates in step (b) and by screening for activity on these two or more peptide substrates in comparison. This aspect is referred to as the "comparison approach".

In a preferred embodiment of this aspect of the invention, the two or more peptide substrates provided in step (b) are linked to different marker molecules, thereby enabling the detection of the cleavage of the two or more peptide substrates consecutively or in parallel. In a particularly preferred embodiment of the invention, two peptide substrates are provided in step (b), one peptide substrate having an amino-acid sequence identical to or resembling the first peptide substrate thereby enabling to monitor the original activity of the first proteases, and the other peptide substrate having an amino-acid sequence identical to or resembling the target substrate sequence thereby enabling to monitor the activity on the target substrate. In an especially preferred embodiment of the invention, these two peptide substrates are linked to fluorescent marker molecules, and the fluorescence properties of the two peptide substrates are sufficiently different in order to distinguish both activities when measured consecutively or in parallel. For example, a fusion protein comprising a first autofluorescent protein, a peptide, and a second autofluorescent protein according to patent application WO 0212543 can be used for this purpose. Alternatively, fluorophores such as rhodamines are linked chemically to the peptide substrates.

In a third aspect of the invention, proteases that recognize and cleave the target sequence preferably are identified by providing in step (b) one or more peptide substrates resembling the target peptide together with competing peptide substrates in high excess. Screening with respect to activity on the substrates resembling the target substrate is then done in the presence of the competing substrates. Proteases having a specificity which corresponds qualitatively to the target specificity, but having only a low quantitative specificity are identified as negative samples in such a screen. Whereas proteases having a specificity which corresponds qualitatively and quantitatively to the target specificity are identified positively. This aspect is referred to as the "competitor approach".

In a preferred embodiment of this aspect of the invention, the one or more peptide substrates resembling the target substrate are linked to marker molecules, thereby enabling the detection of their hydrolysis, whereas the competing peptide substrates do not carry marker molecules. The competing peptide substrates have an amino-acid sequence identical to or resembling the first peptide substrate, or have random amino-acid sequences, thereby acting as competitive inhibitors for the hydrolysis of the marker-carrying peptide substrates.

In embodiment (3) of the invention, proteases that recognize and cleave the target sequence preferably are identified by using intermediate substrates for evolving the protease towards the target substrate specificity. This aspect is hereinafter also referred to as the "intermediate approach". In a first variant of this aspect of the invention, this is achieved by providing in different cycles different peptide substrates, whereby each peptide substrate has an intermediate character with regard to the cycle before and the target peptide substrate.

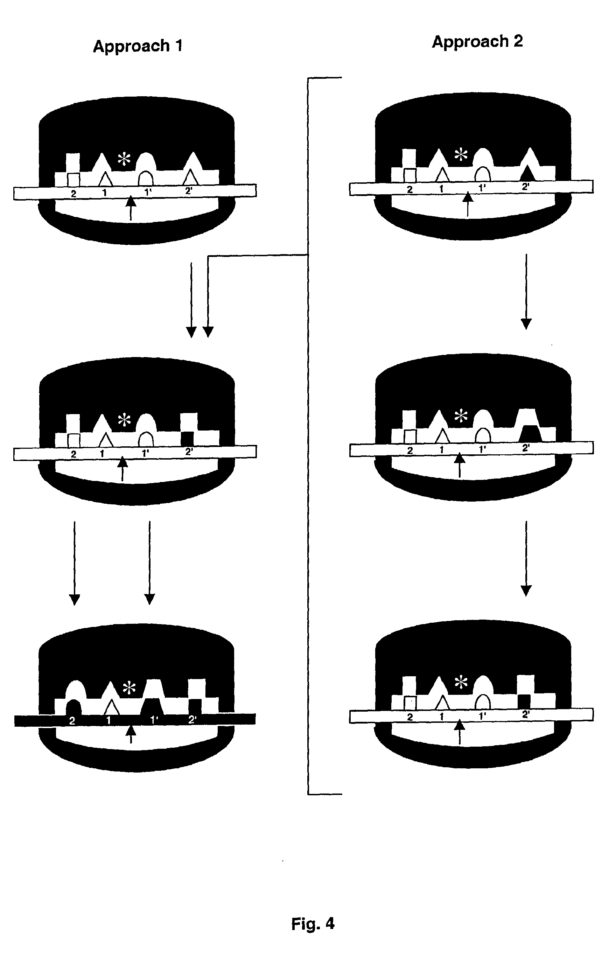

According to this variant, proteases are evolved gradually toward the target specificity. Figure 4 depicts schematically the basic principle of this variant of the intermediate approach.

More generally, in a first variant of this embodiment of the invention is directed to a method for generating sequence-specific proteases with a target substrate specificity, wherein the following steps are carried out:

## EP 1 361 284 A1

(a) providing a population of proteases, wherein each variant is related to one or more first proteases, these first proteases having specificity for a spectrum of peptide substrates or a single peptide substrate;

(b) providing one or more peptide substrates that has an intermediate character with regard to the first peptide substrate and the target substrate;

(c) selecting one or more protease variants from the population of proteases provided in step (a) with respect to their specificity for the substrate provided in step (b);

(d) repeating steps (a) to (c) until one or more protease variants with activity for the intermediate substrate provided in step (b) are identified;

(e) replacing the first peptide substrate in steps (a) and (b) with the intermediate substrate, and the first proteases in step (a) with the protease variants selected in step (c);

and repeating steps (a) to (e) until one or more protease variants with the target substrate specificity are identified.

In this first variant of this aspect of the invention, evolution of protease specificity is directed via consecutive selection on a certain number of intermediate peptide substrates, whereby every peptide substrate resembles more and more the target peptide sequence. This approach is based on the finding that proteases which accept related substrates are usually also related to each other. Relatedness of proteases in the context of this invention is a measure for the homology in the amino acid sequences of two or more enzymes. Moreover, this approach is based on the surprising discovery, that distinguishable subsites in a protease active site can be evolved separately, and that their molecular structure can be attributed to different residues of a peptide substrate (Schlechter & Berger, Biochem. Biophys. Res. Commun. 27 (1967) 157-162).

Intermediate substrates can be realized by substituting amino acid residues at one or more positions from the first peptide sequence with amino acid residues at the same positions from the target peptide sequence. Such intermediates are referred to as "amino acid composition intermediates". Additionally, an intermediate peptide substrate can include one or more amino acid residues at one ore more positions which are neither the residues of the first peptide sequence nor the residues of the target peptide sequence at that position, but are amino acid residues with an intermediate character with respect to the residues in the first and the target substrate. Such intermediates are referred to as "amino acid property intermediates". The intermediate character of this kind of intermediates can be based on one or more physical and chemical parameters, such as the surface of the residue, its volume, the isoelectric point, the side chain pKa, the polarity, or the hydrophobicity. In the following table, the twenty naturally occurring amino acid residues are classified according to these parameters.

Table III:

| Classification of the 20 naturally occurring amino acid residues | | | | | | | |
|---|---|---|---|---|---|---|---|
| Amino acid residue | | Type | Surface [a] [$\text{Å}^2$] | Volume [b] [$\text{Å}^3$] | pI [c] [25°C] | Side chain $pK_a$ [d] | Relative Hydrophobicity [e] |
| A | Ala | aliphatic | 115 | 88.6 | 6.107 | - | 0.62 |
| C | Cys | aliphatic | 135 | 108.5 | 5.02 | 9.1-9.5 | 0.68 |
| D | Asp | aliphatic | 150 | 111.1 | 2.98 | 4.5 | 0.03 |
| E | Glu | aliphatic | 190 | 138.4 | 3.08 | 4.6 | 0.04 |
| F | Phe | aromatic | 210 | 189.9 | 5.91 | - | 1.00 |
| G | Gly | aliphatic | 75 | 60.1 | 6.064 | - | 0.50 |
| H | His | aliphatic | 195 | 153.2 | 7.64 | 6.2 | 0.17 |
| I | Ile | aliphatic | 175 | 166.7 | 6.038 | - | 0.94 |
| K | Lys | aliphatic | 200 | 168.6 | 9.47 | 10.4 | 0.28 |
| L | Leu | aliphatic | 170 | 166.7 | 6.036 | - | 0.94 |
| M | Met | aliphatic | 185 | 162.9 | 5.74 | - | 0.74 |
| N | Asn | aliphatic | 160 | 114.1 | 5.41 | - | 0.24 |

[a] Chothia, C., J. Mol. Biol., 105 (1975) 1-14; [b] Zamyatin, A.A., Prog. Biophys. Mol. Biol., 24 (1972) 107-123; [c] The Merck Index, Merck & Co. Inc., Nahway, N.J., 11(1989); CRC Handbook of Chem.& Phys., Cleveland, Ohio, 58 (1977); [d] Tanford, C., Adv. Prot. Chem., 17 (1962) 69-165; [e] Black, S.D, Mould, D.R, Anal. Biochem., 193 (1991) 72-82

Table III: (continued)

| Classification of the 20 naturally occurring amino acid residues | | | | | | | |
|---|---|---|---|---|---|---|---|
| Amino acid residue | | Type | Surface [a] $[Å^2]$ | Volume [b] $[Å^3]$ | pI [c] $[25°C]$ | Side chain $pK_a$ [d] | Relative Hydrophobicity [e] |
| P | Pro | aliphatic | 145 | 112.7 | 6.3 | - | 0.71 |
| Q | Gln | aliphatic | 180 | 143.8 | 5.65 | - | 0.25 |
| R | Arg | aliphatic | 225 | 173.4 | 10.76 | $\sim$ 12 | 0.00 |
| S | Ser | aliphatic | 115 | 89.0 | 5.68 | - | 0.36 |
| T | Thr | aliphatic | 140 | 116.1 | 5.64 | - | 0.45 |
| V | Val | aliphatic | 155 | 140.0 | 6.002 | - | 0.83 |
| W | Trp | aromatic | 255 | 227.8 | 5.88 | - | 0.88 |
| Y | Tyr | aromatic | 230 | 193.6 | 5.63 | 9.7 | 0.88 |

[a] Chothia, C., J. Mol. Biol., 105 (1975) 1-14; [b] Zamyatin, A.A., Prog. Biophys. Mol. Biol., 24 (1972) 107-123; [c] The Merck Index, Merck & Co. Inc., Nahway, N.J., 11(1989); CRC Handbook of Chem.& Phys., Cleveland, Ohio, 58 (1977); [d] Tanford, C., Adv. Prot. Chem., 17 (1962) 69-165; [e] Black, S.D, Mould, D.R, Anal. Biochem., 193 (1991) 72-82

If, for example, the first substrate were ALY and the target substrate were NRF, intermediate substrates with regard to the amino acid composition would be, for example, ALF, NRY, or ARF (Modifications with regard to the first substrate are indicated). An intermediate substrate with regard to amino acid properties would be, for example, AQF, with the glutamine residue resembling the original leucine with respect to the isoelectric point, but resembling more the arginine residue of the target substrate with respect to hydrophobicity.

Furthermore, the number of consecutive peptide substrates to be used depends on the relatedness of the first peptide sequence and the target peptide sequence as well as the quantitative specificity of the one or more first proteases. The more unrelated the first peptide sequence and the target peptide sequence are, and the higher the specificity of the one or more first proteases is, the more consecutive intermediate peptide substrates are required.

In a preferred embodiment the intermediate peptide substrates provided in step (b) alter from the first peptide substrates at least in that degree to obtain a significant change in the activity of first protease. Preferably, a significant change in activity corresponds to a decrease of 50 percent of the activity referred to the first protease. By analogy, the intermediate peptide substrate in the degree to obtain a significant change of a at least 50 percent decrease of activity referred to the preceding intermediate peptide substrates.

In a more preferred embodiment, the differences as described before are performed by an exchange of 1 to 5 amino acid residues, preferably of 1 to 3 amino acids residues within the specific amino acid sequence (within a specific amino acid sequence recognized by a protease having generally up to about 20 amino acid residues.

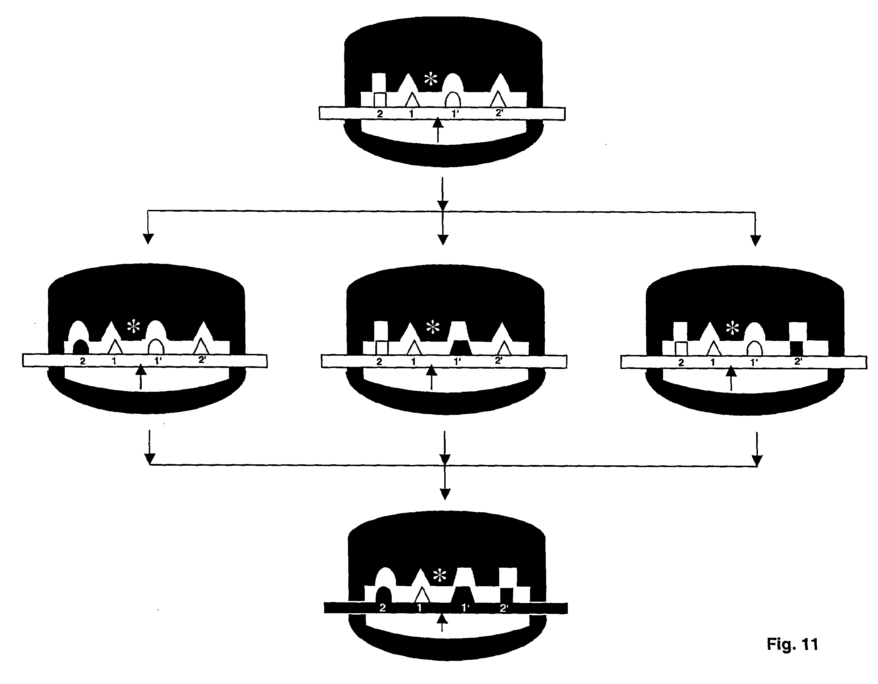

In a second variant of this aspect of the invention, different proteases that have specificity for different intermediates are selected in parallel in a first step of the method. In a second step, proteases which have the target specificity are then selected from a population containing randomly recombined chimeras of the proteases selected in the first step. Preferably, the recombination of different proteases selected in parallel is achieved by employing an in-vitro homologous recombination technique, such as the Recombination Chain Reaction described in patent application WO 0134835. Both forms of intermediates can be used for this variant. However, amino acid composition intermediates are preferably employed. Figure 11 shows schematically the basic principle of this variant of the fourth aspect of the invention.

The different intermediates employed in the first step of this variant are preferably chosen in a way, that the sum of all modifications introduced into these intermediates equals or resembles the characteristics of the target substrate. As an example, when the first substrate were ALY and the target substrate were NRF, suitable amino acid composition intermediates for this embodiment would be NLY, ARY, and ALF. Proteases having specificity for these three substrates would then be randomly recombined and screened for specificity towards the target substrate of this example NRF. Other examples, including intermediates who more than one modification are to be constructed analogously.

In further aspects of the invention, the different aspects mentioned above are combined with each other. In a preferred combination, screening for proteases with decreased Michaelis-Menten constants is combined with the use of intermediate substrates. In another preferred embodiment, screening for proteases with decreased Michaelis-Menten constants is combined with the screening of two or more substrates consecutive or in parallel. In further preferred embodiment, screening for proteases with decreased Michaelis-Menten constants is combined with using an excess of competing, unlabelled substrate. In a particularly preferred embodiment, the three aspects, screening for proteases with decreased Michaelis-Menten constants, screening of two or more substrates in parallel, and the use of intermediate substrates, are combined.

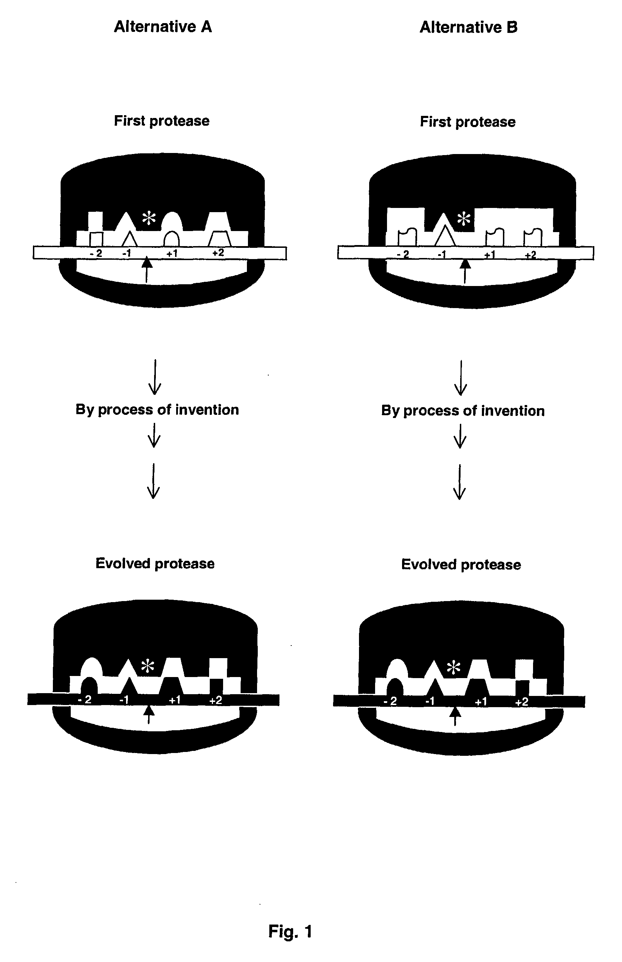

<u>Figure 1</u> depicts schematically the two alternatives A and B of the method of the invention. Starting with a first protease, the aim of the invention is the generation of an evolved protease with a high specificity for a target peptide substrate which is characterized by its amino acid sequence.

For the purpose of this figure, different shapes represent different amino acid residues, and the inverse profile of the shapes represent the protease's recognition sites, respectively. Shapes with a swung tilde at the top represent any amino acid residue at that position. The active site of the enzyme is indicated by an asterisk, and the arrow indicates the cleavage site within the substrate.

The type of the one or more proteases used as first proteases defines whether alternative A or alternative B is to be employed. In alternative A, the first protease is characterized by an already high specificity towards a defined, first substrate. According to the method of the invention, this specificity has to be changed qualitatively into the target specificity. In alternative B, the first protease has a relatively low specificity, i.e. it does not discriminate between a pool of substrates that differs, for example, at positions P2, P1', and/or P2'. By the process of the invention, only the quantitative specificity of those proteases is increased towards the value of the target specificity.

<u>Figure 2</u> distinguishes the two alternatives A and B of the method of the invention by showing schematically the qualitative and quantitative changes in specificity during evolution towards the target specificity. The quantitative specificity s, as defined in the framework of this invention, refers to the ratio between all accepted and all possible substrates. The qualitative specificity refers to the amino acid composition and sequence of accepted substrates. Specificities of the first proteases (open circles) and the evolved protease (filled circle) are indicated schematically. In alternative A, the first protease has a quantitative specificity in the range of the target specificity, but a qualitative specificity that differs from the target specificity. In order to generate the target specificity, the specificity is changed qualitatively only. In alternative B, the first protease has the qualitative specificity of the target substrate, but a quantitative specificity that is far below the target specificity. In order to generate the target specificity, the specificity is changed quantitatively only. When both alternatives are combined, the first protease has neither the qualitative nor the quantitative specificity of the target substrate. In order to generate the target specificity, the specificity is changed quantitatively and qualitatively.

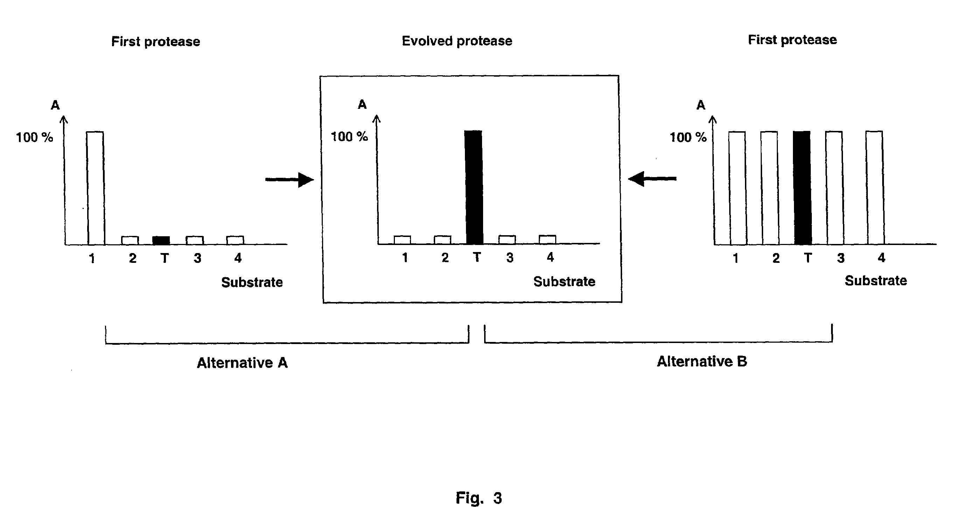

<u>Figure 3</u> illustrates schematically how proteases with changed catalytic activities are evolved using the two alternatives A and B of the method of the invention.

According to the method of the invention, the catalytic activity (abbreviated as A) can be used as a selection parameter. In alternative A, the first protease hydrolyses only substrate 1, whereas other substrates including the target substrate (T) are not or only very slowly hydrolyzed. By use of the method of the invention, proteases are evolved that hydrolyze specifically the target substrate, whereas other substrates including the first substrate are not or only very slowly hydrolyzed. These evolved proteases are selected by an increase of catalytic activity on the target substrate and a decrease of catalytic activity on the first substrate (comparison approach). Alternatively, selection can be based on the affinity towards the target substrate (affinity approach). In alternative B, the first protease hydrolyzes all substrates including the target substrate (T). By use of the method of the invention, proteases are evolved that hydrolyze specifically the target substrate, whereas other substrates including the first substrate are not or only very slowly hydrolyzed.

These proteases are selected by screening with an excess of competing substrates (competitor approach) or by screening for higher substrate affinity (affinity approach). In general, the evolved protease can be identified by the comparison of the catalytic activity towards offered substrates including the first substrates and the target substrate.

<u>Figure 4</u> depicts schematically in two different forms the intermediate approach as one particular aspect of the invention. For description of symbols, refer to figure 1. The intermediate approach uses one or more intermediate substrates to guide the evolution of specificity gradually towards the target specificity in steps as small as necessary. Intermediate substrates are substrates that have an intermediate character when compared with the first and the target substrate. Intermediates can be classified into two forms. First, intermediate substrates can be provided by replacing at least one but less than all amino acid residues of the first substrate with amino acid residues from the target substrate (Intermediate with respect to amino acid composition, Approach 1). Secondly, intermediate substrates can be provided by selectively introducing at defined positions of the substrate amino acid residues whose properties range between those of the corresponding amino acid residue in the first and the target substrate (Intermediate with respect to amino acid properties, Approach 2). As a further alternative, both intermediate approaches can be combined. Preferably, as shown in the figure, the second approach is implemented into the first approach whenever the step between two intermediates is too large.

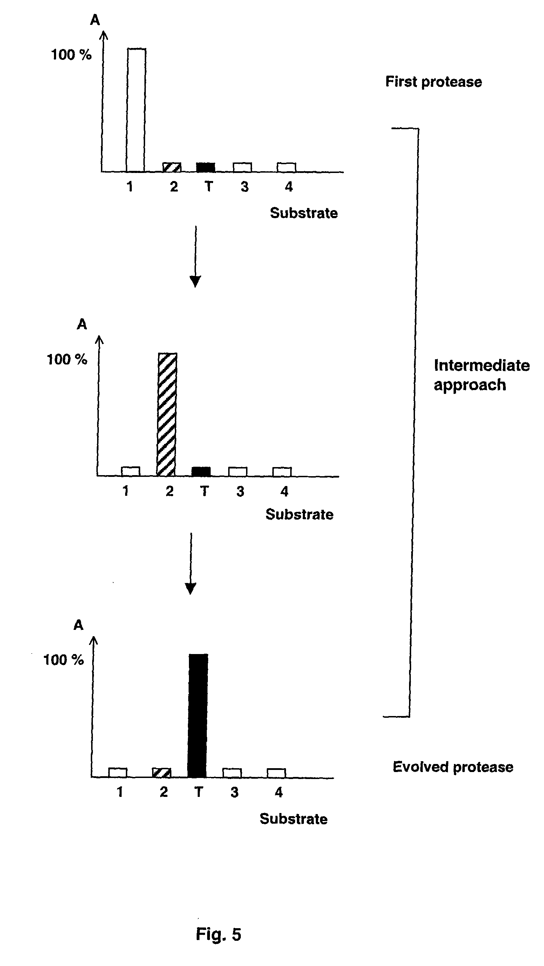

<u>Figure 5</u> illustrates schematically how, according to the invention, proteases with changed catalytic activities are evolved using the intermediate approach. The first protease has a high activity on a first substrate (1) and no or very low activity on all other substrates including the target substrate (T). The following essential step is the provision of an intermediate substrate (2) as illustrated in Figure 4 . By screening for catalytic activity on this substrate, protease variants with an increased activity on this intermediate substrate are selected. This intermediate step can be repeated with a gradual variation of the intermediate substrate towards the nature of the target substrate, until an evolved protease is isolated which shows catalytic activity to the target substrate only and no or very low activity on the first substrate and other substrates.

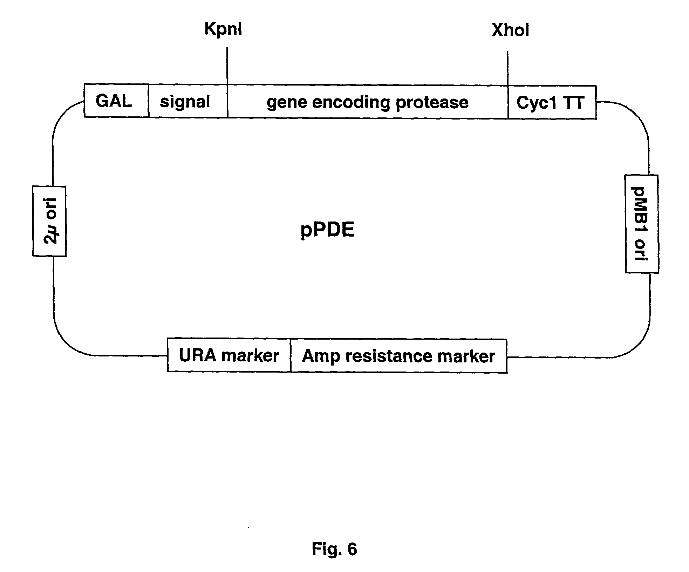

Figure 6 shows schematically the shuttle vector pPDE that can be used for the method of the invention. The vector comprises a S. cerevisiae origin (2μ ori), an E. coli origin (pMB1 ori), a S. cerevisiae marker (URA3), an E. coli marker (AmpR), and the expression cassette which is composed of a galactose-inducible S. cerevisiae promotor (GAL), a signal sequence for secretion of the expressed protein (signal), a KpnI and an XhoI recognition site for inserting the gene of interest, and a terminator (Cyc1).

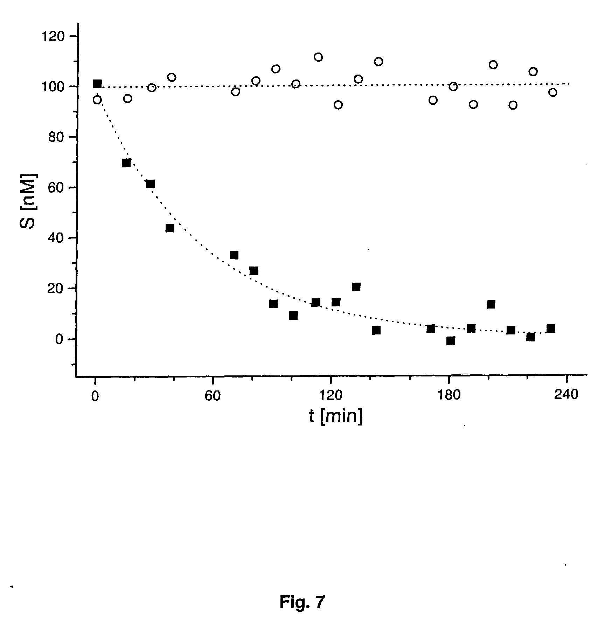

Figure 7 shows exemplarily the hydrolysis of a peptide substrate catalyzed by the tobacco etch virus protease monitored by cross-correlation confocal fluorescence spectroscopy (cc-FCS). The peptide substrate with the sequence ENLYFQS is specifically recognized and hydrolyzed by the TEV protease. 100 nM double-labeled peptide (Alexa 488, Cy5) were incubated with (filled squares) and without (open circles) addition of 0.01 U/μl protease in assay buffer containing 50 mM Tris-HCl pH 8.0, 0.5 mM EDTA, 10 mM DTT, 0.05% glycerol.

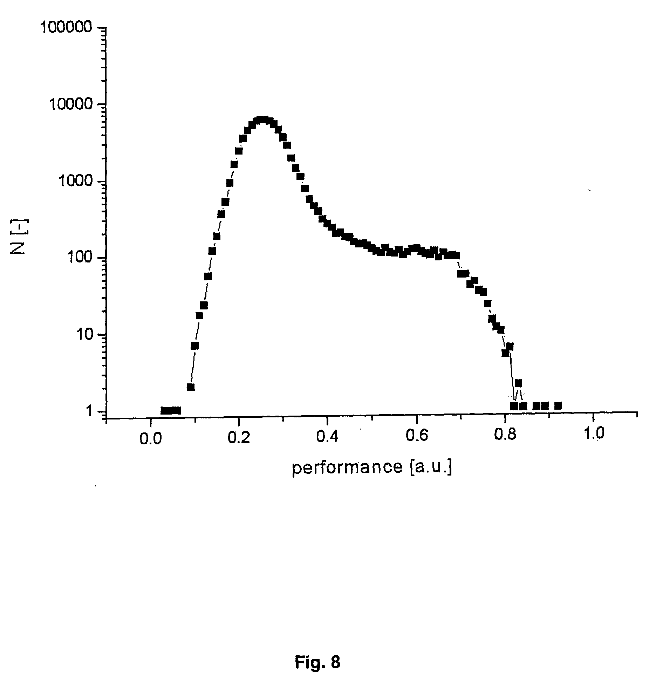

Figure 8 shows exemplarily a distribution of catalytic activities obtained by screening a population of protease variants on the substrate WLGLVPGG (intermediate 1, see Example VI) using confocal fluorescence spectroscopy. Shown is the frequency N with which a certain catalytic activity (performance, arbitrary units) is identified. Low values represent low catalytic activities, whereas high values represent high catalytic activities on the substrate. Genes encoding variants having highest performance values are isolated and evaluated with respect to their specificity. These variants are then used as first proteases for the next cycle. This procedure is repeated until there are protease variants identified that have the target specificity.

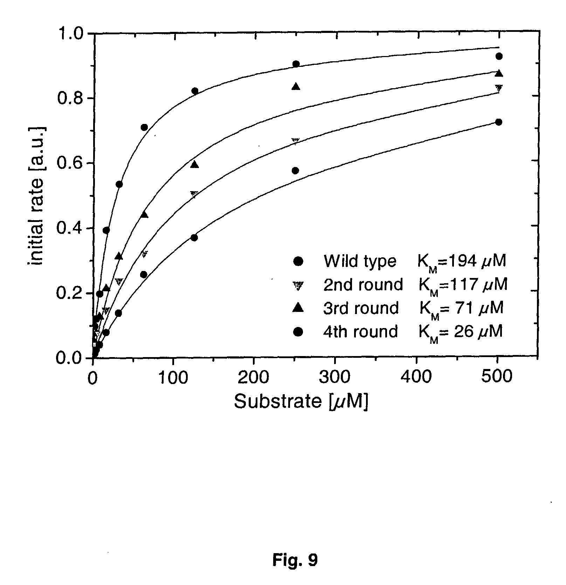

Figure 9 shows exemplarily the decrease in $K_M$ during evolution toward higher affinity using the affinity approach of the invention. The protease used as first protease (wild type) in this experiment was subtilisin E from B. subtilis which had a $K_M$ of 194 μM. This $K_M$ was gradually decreased by use of the method of the invention by a factor of 7.5 down to 26 μM.

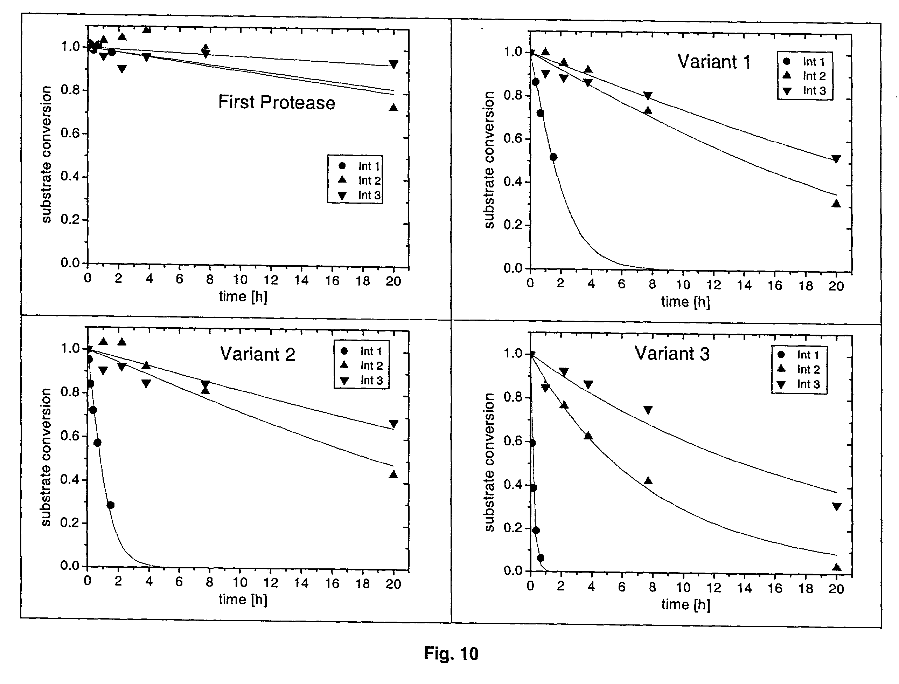

Figure 10 shows exemplarily the change in specificity during evolution of proteases towards the specificity of t-PA. The activity of variants 1, 2, and 3 were evaluated using the substrates intermediate 1, intermediate 2, and intermediate 3 of example VI. The decrease in the substrate concentration corresponds to proteolytic activity. The faster this decrease is, the higher is the catalytic activity of the protease variant. While the first protease has very low activity on intermediate 1, and no activity on intermediates 2 or 3, the evolved variants show various activities on the three intermediate substrates.

Figure 11 depicts schematically a preferred variant of the intermediate approach of the invention, where proteases are in a first step selected according to their specificity for different intermediate substrates in parallel. Protease are then selected according their specificity for the target substrate from a population containing recombined variants of the protease variants selected in the first step.

The invention is further explained by the following Examples. It is understood that the examples and embodiments described therein are for illustrative purposes only and that various modifications or changes in light thereof will be suggested to persons skilled in the art and are to included within the spirit and purview of this application and are considered within the scope of the appended claims. All publications, patents, and patent applications cited herein are hereby incorporated by reference in their entirety for all purposes.

**Examples**

In the following examples, materials and methods of the present invention are provided including the determination of catalytic properties of enzymes obtained by the method. It should be understood that these examples are for illustrative purpose only and are not to be construed as limiting this invention in any manner.

In the experimental examples described below, standard techniques of recombination DNA technology were used that were described in various publications, e.g. Sambrook et al. (1989), Molecular Cloning: A Laboratory Manual, Cold Spring Harbor Laboratory, or Ausubel et al. (1987), Current Protocols in Molecular Biology 1987-1988, Wiley Interscience, Methods in Yeast Genetics (1994) A Cold Spring Harbour Laboratory Manual, which are incorporated herein in their entirety by reference. Unless otherwise indicated, restriction enzymes, polymerases and other enzymes as well as DNA purification kits were used according to the manufacturers specifications.

Example I: Molecular cloning of genes encoding protease variants

Genes encoding protease variants were cloned into a vector suitable for extracellular expression of proteins by the yeast Saccharomyces cerevisiae. The vector used is a derivate of the plasmid pYES2, which is commercially available from Invitrogen, Inc. A map of the plasmid is shown in Figure 6. The vector contains a 2μ origin for amplification in S. cerevisiae, a pMB1 origin for amplification in E. coli, a URA marker for selection in S. cerevisiae, an ampicillin resistance marker for selection in E. coli, as well as a GAL promoter and a Cyc1 transcription terminator for inducible expression in S. cerevisiae. A 90 bp fragment that contains the leader sequence encoding the signal peptide from the BAR1 gene of S. cerevisiae was introduced behind the GAL1 promoter. Restriction sites KpnI and XhoI served as insertion sites for heterelogous genes to be expressed. Cloning of genes encoding protease variants was done as follows: the coding sequence of the mature protein was amplified by PCR using primers that introduced a KpnI site at the 5' end and a XhoI site at the 3' end.

This PCR fragment was cloned into the appropriate sites of the vector and identity was confirmed by sequencing.

Example II: Providing populations of protease variants

A population of protease variants was provided by random modification of genes encoding proteases with known substrate specificities, followed by expression of the protease variants encoded by these modified genes using S. cerevisiae as a suitable host organism. First, genes encoding protease variants with known substrate specificities were PCR amplified under error-prone conditions, essentially as decribed by Cadwell, R.C and Joyce, G.F. (PCR Methods Appl. 2 (1992) 28-33). Error-prone PCR was done using 30 pmol of each primer, 20 nmol dGTP and dATP, 100 nmol dCTP and dTTP, 20 fmol template, and 5 U Taq DNA polymerase in 10 mM Tris HCl pH 7.6, 50 mM KCl, 7 mM $MgCl_2$, 0.5 mM $MnCl_2$, and 0.01 % gelatin for 20 cycles of 1 min at 94°C, 1 min at 65°C and 1 min at 72°C. The resulting DNA library was purified using the Qiaquick PCR Purification Kit following the suppliers' instructions. PCR products were digested with restriction enzymes *Xho*I and *Kpn*I and purified as described in Example I. Afterwards, the PCR products were ligated into the vector which was digested with XhoI and KpnI, gel-purified and dephosphorylated. The ligation products were transformed into E. coli, amplified in LB containing ampicillin as marker, and the plasmids were purified using the Qiagen Plasmid Purification Kit following the suppliers' instructions. Resulting plasmids were transformed into S. cerevisiae cells. Populations of protease variants were provided by inducing expression in the transformed S. cerevisiae cells by adding 2% galactose to the medium. Alternatively, genes encoding protease variants with known substrate specificities were statistically recombined at homologous positions by use of the Recombination Chain Reaction, essentially as described in WO 0134835. PCR products of the genes encoding the protease variants were purified using the QIAquick PCR Purification Kit following the suppliers' instructions, checked for correct size by agarose gel electrophoresis and mixed together in equimolar amounts. 80 μg of this PCR mix in 150 mM TrisHCL pH 7.6, 6.6 mM $MgCl_2$ were heated for 5 min at 94 °C and subsequently cooled down to 37 °C at 0.05 °C/sec in order to re-anneal strands and thereby produce heteroduplices in a stochastic manner. Then, 2.5 U Exonuclease III per μg DNA were added and incubated for 20, 40 or 60 min at 37 °C in order to digest different lengths from both 3' ends of the heteroduplices. The partly digested PCR products were refilled with 0.6 U Pfu polymerase per μg DNA by incubating for 15 min at 72 °C in 0.17 mM dNTPs and Pfu polymerase buffer according to the suppliers' instructions. After performing a single PCR cycle, the resulting DNA was purified using the QIAquick PCR Purification Kit following the suppliers' instructions, digested with KpnI and XhoI and ligated into the linearized vector. The ligation products were transformed into E. coli, amplified in LB containing ampicillin as marker, and the plasmids were purified using the Qiagen Plasmid Purification Kit following the suppliers' instructions. Resulting plasmids were transformed into S. cerevisiae cells. Populations of protease variants were provided by inducing expression in the transformed S. cerevisiae cells by adding 2 % galactose to the medium.

Example III: Providing peptide substrates that resemble the target substrate

All peptide substrates were synthesized on a peptide synthesizer using the approach of Merrifield et al. (Nature. 207 (1965) 522-523). Peptide substrates that resemble the target substrate were designed by substituting the amino acid residues at one or more positions of the first peptide substrate with the amino acid residues at the one or more positions of the target substrate. Alternatively, the amino acid residues at one or more positions of the first peptide substrate were substituted with amino acid residues that have an intermediate character with respect to the amino acid residues of the first peptide substrate and the amino acid residues of the target peptide substrate. For the determination of the intermediate character of amino acid residues refer to Table III. Marker fluorophores were attached to the peptide substrates either via the amino group of the N-terminus or via the carboxy group of the C-terminus. Alternatively, a cysteine residue was added either at the N-terminus or at the C-terminus of the peptide, and the marker fluorophor was chemically attached to the thiol group of the cysteine residue.
Alexa 488 (Molecular Probes Inc., Oregon, USA) and Cy-5 (Amersham Biosciences Europe GmbH, Freiburg, Germany) were typically used as fluorophor markers. Protease cleavage of the peptide substrate was monitored by cross-correlation FCS (Proc.Natl.Acad.Sci.USA. 95 (1998) 1416-1420). As an example, the cleavage of a peptide substrate that contains the target substrate for tobacco etch virus protease (TEV protease) and has the Alexa 488 fluorophor attached to the C-terminus of the peptide and the Cy-5 fluorophor attached to the N-terminus of the peptide is shown in Figure 7. The TEV protease has already a relatively high specificity (s = 4.9, see Table I). Cleavage was done at a peptide concentration of 100 nM by adding 0.01 U/µl TEV protease in assay buffer containing 50 mM Tris-HCl pH 8.0, 0.5 mM EDTA, 10 mM DTT, and 0.05% glycerol.

Example IV: Screening procedure

In order to identify enzyme variants having the desired substrate specificity, a screening approach based on a confocal fluorescence spectroscopy set-up as disclosed in WO 9416313 was used. Either the cell suspension of a S. cerevisiae culture directly, or an aliquot of the cell-free supernatant was used as the sample containing the secreted protease variant. After adding the substrate to the sample and incubation for a certain period of time, the samples were subjected to measurement by confocal fluorescence spectroscopy. If necessary, this procedure was repeated several times in order to measure kinetics of the proteolytic cleavage. Consequently, the samples were ranked according to proteolytic activity, and samples exceeding a certain activity threshold were identified in order to isolate the gene encoding the corresponding protease variant. The distribution of proteolytic activities of protease variants obtained by this procedure is shown in Figure 8.

Example V: Generating sequence-specific proteases with increased affinity towards the target peptide substrate by screening at low substrate concentrations

Protease variants that have an increased affinity towards the target peptide substrate were generated by the method of the invention based on screening at low substrate concentrations. By means of error-prone PCR (according to Cadwell, R.C and Joyce, G.F., PCR Methods Appl. 2 (1992) 28-33), a population of protease variants was generated that is related to the alkaline protease subtilisin E from Bacillus subtilis, which has a relatively low specificity (s = 0.82). This correlates to the relatively high $K_M$ which is in the range of 150- 200 µM. The population of protease variants was screened at a complexity of $10^6$ variants by confocal fluorescence spectroscopy employing substrate concentrations in the range of 10 nM. Variants isolated in this first screen were used as first proteases in a second cycle to provide another population of protease variants. Analogously, variants isolated in subsequent cycles were used as first proteases in the following cycle. The population of variants provided in the second cycle and all subsequent cycles was generated by a combination of error-prone PCR (see above) and in-vitro homologous recombination (according to WO 0134835). Variants isolated from the first four cycles of this procedure were analyzed kinetically. The increase in affinity towards the substrate over the four rounds corresponds to the decrease in $K_M$ of the best performers of each cycle which is shown in Figure 9.

Example VI: Generating sequence-specific proteases with a target substrate specificity resembling the specificity of tissue-type plasminogen activator

Proteases were generated by the method of the invention that had a specificity that was altered towards the specificity of tissue-type plasminogen activator (t-PA). The BAR1 protease from Saccharomyces cerevisiae was used as first protease. This protease belongs to the group of aspartic proteinases (Mac Kay et al.; Structure an Function of the Aspartic Proteinases (1991) 161-172). It is specific for peptide substrates containing the amino acid sequence WLQLKPGQ, and catalyses the cleavage at the peptide bond between the second leucine and the lysine residue. Populations of protease variants that were related to the BAR 1 protease or proteases isolated in subsequent screening cycles were generated by means of error-prone PCR (according to Cadwell, R.C and Joyce, G.F., PCR Methods Appl. 2 (1992) 28-33) and in-vitro homologous recombination using the Recombination Chain Reaction (WO 0134835). Protease variants were screened for proteolytic activity at complexities of $10^6$ variants by confocal fluorescence spectroscopy. The BAR 1 protease used as first protease already had a relatively high specificity which was in the range of the target specificity. Therefore, a combination of the affinity approach and of the intermediate approach was used. Screening at low concentrations kept the specificity of the protease high, while screening on intermediate substrates enabled the evolution towards the new specificity. Three intermediate substrates were constructed. Intermediate substrate 1 had the amino acid sequence WLGLVPGG, intermediate substrate 2 the amino acid sequence WLGQVPGG, and intermediate substrate 3 the amino acid sequence WLGRVPGG. The target substrate specificity of t-PA is directed to CPGRVVGG with cleavage between the arginine residue and the first valine residue. All substrates are shown in Table IV.

## Table IV

P4 P3 P2 P1 P1'P2'P3' P4'

First substrate  W L Q L K P G Q

Intermediate 1  W L G L V P G G

Intermediate 2  W L G ▨ V P G G

Intermediate 3  W L G R V P G G

Target substrate  C P G R V V G G

Intermediate 1 was an amino acid composition intermediate due to the fact that it contained at positions P4, P3, P1 and P2' the same amino acid residues as the first substrate, and at positions P2, P1', and P4' the same residues as the target substrate. Intermediate 2 was an amino acid property intermediate with regard to intermediate 1 and the target substrate. It resembled intermediate 1 but contained at position P1 an glutamine residue which has an intermediate character compared to the leucine residue present at that position in the first substrate and the arginine residue present at that position in the target substrate. Intermediate 3 as another amino acid composition intermediate was based on amino acid residues stemming from both, the first substrate and the target substrate, as intermediate substrate 1 does, but, in contrast to the latter one, shared one additional position with the target substrate. The changed specificities of different variants that were generated by this method are shown in Figure 10.

**Claims**

1. A method for generating sequence-specific proteases with target substrate specificities which comprises the following steps

   (d) providing a population of proteases comprised of variants of one first protease or of variants or chimeras of two or more first proteases, said first proteases having a substrate specificity for a particular amino acid sequence of a first peptide substrate;
   (e) contacting said population of proteases with one or more second substrates, comprising at least one specific amino acid sequence resembling the amino acid sequence of the target peptide substrate but being not present within the first peptide substrate; and
   (f) selecting one or more protease variants from the population of proteases provided in step (a) having specificity for said specific amino acid sequence of the second substrates provided in step (b) under conditions that allow identification of proteases that recognize and hydrolyse preferably said specific one amino acid sequence within the second substrates.

2. The method of claim 1, wherein the selection conditions in step (c) are achieved by

   (i) screening for protease activity under low substrate concentrations, thereby increasing affinity for the second substrate,
   (ii) screening for protease activity by using two or more substrates in comparison, thereby increasing the selectivity of the enzyme,
   (iii) screening for protease activity by adding in excess peptides other than the second peptide, thereby using the added peptides as competitors, or

(iv) any combination thereof.

3. The method of claim 1 or 2, wherein steps (a) to (c) are repeated cyclically until one or more protease variants with specificity for the second substrate are identified, and wherein protease variants selected in one cycle are used as first proteases in the following cycle, and wherein at least one cycle and less than 100

4. The method according to any one of claims 1 to 3, wherein only one second substrate is used in the one or more cycles, and wherein the second substrate is identical with the target substrate.

5. The method according to any one of claims 1 to 3, wherein different second substrates are used, and wherein the second substrates have an intermediate character with regard to the first substrate and the target substrate, and wherein the last second substrate that is used is identical with the target substrate.

6. The method of claim 5, wherein different second substrates are used in consecutive cycles, and wherein each second substrate has intermediate character with regard to the second substrate used before and the target substrate.

7. The method of claim 5 or 6, whereby in at least one cycle steps (b) to (c) are executed with different second substrates in parallel, and wherein the protease variants isolated in such a parallel way are combined and used as first proteases in the next cycle.

8. The method according to any of claim 5 to 7, wherein the intermediate character of the intermediate substrates is based on

   (i) the amino acid composition,
   (ii) the amino acid sequence,
   (iii) the physical and/or the chemical properties of the amino acid residues within the specific amino acid sequence, whereby preferably one or more properties from the group consisting of the following amino acid properties is used: the surface, the volume, the isoelectric point, the side chain pKa, the charge, the polarity, the hydrophobicity, or
   (iv) any combination thereof.

9. The method according to any one of claims 1 to 8, wherein the second substrates differ from the first substrates in that 1 to 5, preferably 1 to 3 amino acid residues within the specific amino acid sequence are exchanged.

10. The method according to any one of claims 1 to 9, wherein

   (i) the population of proteases is obtained through random nucleic acid mutagenesis, cassette mutagenesis, site-saturation mutagenesis, homologous *in vitro* recombination, homologous *in-vivo* recombination, non-homologous recombination, or a combination thereof; and/or
   (ii) the expression of the population of proteases is done by use of host cells, preferably from bacterial, yeast, insect, viral or mammalian origin, or is done by use of cell-free protein expression systems, and/or
   (iii) the coupling of protease genotype and phenotype is achieved by use of sample carriers that enable compartment of samples, and the distribution of genotypes into sample carriers is done at a multiplicity per compartment that allows sufficient differentiation of phenotypes.

11. The method according to any one of claims 1 to 10, wherein the second substrates carry functional groups that enable the detection of the hydrolysis of the substrate, said functional groups being

   (i) one or more fluorophores or chromophores, whose spectroscopic properties change upon hydrolysis of the peptide, whereby screening is performed through determination of the change in spectroscopic properties; or
   (ii) two fluorophores which are distinguishable by their fluorescence properties and which are attached to opposite ends of the second substrate, whereby the screening is performed through confocal fluorescence spectroscopy at fluorophore concentrations below 1 $\mu$M; or
   (iii) two fluorophores which form a fluorescence resonance energy transfer (FRET) pair and which are attached to opposite ends of the second substrate, whereby screening is performed through determination of the decrease in the energy transfer between the two fluorophores; or
   (iv) a first and second autofluorescent protein flanking the second substrate, whereby the screening is performed through confocal fluorescence spectroscopy at substrate concentrations below 1 µM; or

(v) a fluorophore and a quencher molecule which are attached to opposite ends of the second substrate, whereby screening is performed through determination of the decrease in quenching of the fluorophore; or

(vi) a fluorophor or a chromophor and a binding moiety which are attached to opposite ends of the second substrate, whereby screening is performed through determination of binding of the binding moiety to a specific binding partner; or

(vii) a radioactive label and a binding moiety which are attached to opposite ends of the second substrate, whereby screening is performed through use of a scintillation proximity assay; or

(viii) any combination thereof.

12. A method according to any of claims 1 to 11, wherein the first protease is selected from the group of proteases consisting of serine proteases, cysteine proteases, aspartic proteases and metalloproteases, and wherein the first protease is preferably selected from the group of proteases consisting of Papain, Bromelain, Trypsin, Pepsin, Chymotrypsin, Subtilisin, SET, Human elastase, Cathepsin, Chymase, Sacharomycopsis fibuligera PEP I, Kallikrein, Urokinase, Thermolysin, Collagenase, Pseudomonas aeruginosa elastase, TEV protease, HIV-1 protease, BAR1 protease, Factor Xa, Thrombin, Tissue-type plasminogen activator, Kex2 protease, TVMV-protease, RSV protease, MuLV protease, MPMV protease, MMTV protease, BLV protease, EIAV protease, SIVmac protease.

13. The method according to any one of claims 1 to 12, preferably according to any one of claims 5 to 12, wherein the target protease has a specificity similar to tissue-type plasminogen activator and cleaves the target substrate CPGR$^\downarrow$VVGG,

14. The method of claim 13, wherein the starting protease is BAR1 protease from *S. cerevisiae* and preferably the following second/intermediate substrates are utilized:

(i) WLGLVPGG
(ii) WLGQVPGG
(iii) WLGRVPGG
(iv) CPGRVVGG.

15. A sequence-specific protease obtainable by the method according to any one of claims 1 to 14, preferably by the method according to claim 12 or 13.

16. The protease of claim 15 which has a specificity similar to tissue-type plasminogen activator, cleaves the target substrate CPGR$^\downarrow$VVGG and is derived from the BAR1 protease from *S. cerevisiae.*

**Alternative A**

**Alternative B**

**First protease**

**First protease**

By process of invention

By process of invention

**Evolved protease**

**Evolved protease**

**Fig. 1**

Quantitative
Specificity

s

high

(S >> 1)

**Alternative A**

**Combination**

**Alternative B**

low

(S ≤ 1)

Qualitative
Specificity

**of First**
**substrate**

**of Target**
**substrate**

**Fig. 2**

Fig. 3

**Approach 1**

**Approach 2**

**Fig. 4**

Fig. 5

Fig. 6

**Fig. 7**

Fig. 8

**Fig. 9**

Fig. 10

Fig. 11

**European Patent Office**

## PARTIAL EUROPEAN SEARCH REPORT

**Application Number**

which under Rule 45 of the European Patent Convention EP 02 01 0576
shall be considered, for the purposes of subsequent
proceedings, as the European search report

### DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| Y | KOLTERMANN ANDRE ET AL: "Principles and methods of evolutionary biotechnology." BIOPHYSICAL CHEMISTRY, vol. 66, no. 2-3, 1997, pages 159-177, XP001085255 ISSN: 0301-4622 see the whole document | 1-14 | C12Q1/37 C12N9/50 |
| Y | WO 99 11801 A (BROAD PETER MICHAEL ;HOLLIS MELVYN (GB); ZENECA LTD (GB); CHARLES) 11 March 1999 (1999-03-11) * page 10, line 3-17 * | 1-14 | |
| A | US 2002/022243 A1 (ELLMAN JONATHAN A ET AL) 21 February 2002 (2002-02-21) —/— | | |

**TECHNICAL FIELDS SEARCHED (Int.Cl.7)**

C12Q

### INCOMPLETE SEARCH

The Search Division considers that the present application, or one or more of its claims, does/do not comply with the EPC to such an extent that a meaningful search into the state of the art cannot be carried out, or can only be carried out partially, for these claims.

Claims searched completely :

Claims searched incompletely :

Claims not searched :

Reason for the limitation of the search:

see sheet C

| Place of search | Date of completion of the search | Examiner |
|---|---|---|
| MUNICH | 3 July 2002 | Grosskopf, R |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

European Patent
Office

**INCOMPLETE SEARCH
SHEET C**

Application Number

EP 02 01 0576

Claim(s) searched completely:
        1-14

Claim(s) not searched:
        15,16

Reason for the limitation of the search:

The protease according to Claim 15 is not defined by any meaningful
technical feature but merely by reference to a broad process for its
isolation. Such a defintionnredners a search for the claimed protease
impossible. The same , in principle, applies for the protease of Claim 16
which is either characterised by open-ended non-limiting expressions
(specificity similar, derived from) or by a desired result to be achieved
(desired target substrate).

**European Patent Office**

## PARTIAL EUROPEAN SEARCH REPORT

Application Number

EP 02 01 0576

| | DOCUMENTS CONSIDERED TO BE RELEVANT | | CLASSIFICATION OF THE APPLICATION (Int.Cl.7) |
|---|---|---|---|
| Category | Citation of document with indication, where appropriate, of relevant passages | Relevant to claim | |
| A | SMITH T A ET AL: "DIRECT SELECTION FOR SEQUENCES ENCODING PROTEASES OF KNOWN SPECIFICITY" PROCEEDINGS OF THE NATIONAL ACADEMY OF SCIENCES OF USA, NATIONAL ACADEMY OF SCIENCE. WASHINGTON, US, vol. 88, June 1991 (1991-06), pages 5159-5162, XP000946644 ISSN: 0027-8424 ----- | | |

TECHNICAL FIELDS SEARCHED (Int.Cl.7)

EPO FORM 1503 03.82 (P04C10)

## ANNEX TO THE EUROPEAN SEARCH REPORT
## ON EUROPEAN PATENT APPLICATION NO.

EP 02 01 0576

This annex lists the patent family members relating to the patent documents cited in the above-mentioned European search report.
The members are as contained in the European Patent Office EDP file on
The European Patent Office is in no way liable for these particulars which are merely given for the purpose of information.

03-07-2002

| Patent document cited in search report | | Publication date | Patent family member(s) | | Publication date |
|---|---|---|---|---|---|
| WO 9911801 | A | 11-03-1999 | EP<br>WO | 1009835 A2<br>9911801 A2 | 21-06-2000<br>11-03-1999 |
| US 2002022243 | A1 | 21-02-2002 | AU<br>WO | 6509201 A<br>0194332 A1 | 17-12-2001<br>13-12-2001 |

EPO FORM P0459

For more details about this annex : see Official Journal of the European Patent Office, No. 12/82

SEQUENCE LISTING

<110> DIREVO Biotech AG

<120> Process for Gernerating Sequence-Specific Proteases by Directed Evolution and Use Thereof

<130> 021250ep/JH/ml

<140>
<141>

<160> 6

<170> PatentIn Ver. 2.1

<210> 1
<211> 8
<212> PRT
<213> Artificial Sequence

<220>
<223> Description of Artificial Sequence: Protease substrate

<400> 1
Trp Leu Gln Leu Lys Pro Gly Gln
 1               5

<210> 2
<211> 8
<212> PRT
<213> Artificial Sequence

<220>
<223> Description of Artificial Sequence: Protease substrate

<400> 2
Trp Leu Gly Leu Val Pro Gly Gly
 1               5

<210> 3
<211> 8
<212> PRT
<213> Artificial Sequence

<220>
<223> Description of Artificial Sequence: Protease substrate

<400> 3
Trp Leu Gly Gln Val Pro Gly Gly
 1               5

<210> 4
<211> 8
<212> PRT
<213> Artificial Sequence

<220>
<223> Description of Artificial Sequence: Protease substrate

```
<400> 4
Trp Leu Gly Arg Val Pro Gly Gly
  1               5


<210> 5
<211> 8
<212> PRT
<213> Artificial Sequence

<220>
<223> Description of Artificial Sequence: Protease
      substrate

<400> 5
Cys Pro Gly Arg Val Val Gly Gly
  1               5


<210> 6
<211> 7
<212> PRT
<213> Artificial Sequence

<220>
<223> Description of Artificial Sequence: TEV protease
      substrate

<400> 6
Glu Asn Leu Tyr Phe Gln Ser
  1               5
```